# EUROPÄISCHE PATENTSCHRIFT

(11) **EP 2 353 647 B1**
(45) Veröffentlichungstag und Bekanntmachung des Hinweises auf die Patenterteilung: **18.01.2017**
(21) Anmeldenummer: 10192867.9
(22) Anmeldetag: 29.11.2010
(51) Int. Cl.: A61N 5/10, A61B 5/055

(54) **Vorrichtung mit einer Kombination aus einer Magnetresonanzvorrichtung und einer Strahlentherapievorrichtung**
Device with a combination of a magnetic resonance device and a radiation therapy device
Dispositif doté d'une combinaison à partir d'un dispositif à résonance magnétique et d'un dispositif de thérapie par rayonnement

(30) Priorität: 10.02.2010 DE 102010001743
(43) Veröffentlichungstag der Anmeldung: 10.08.2011
(73) Patentinhaber: Siemens Healthcare GmbH, 91052 Erlangen (DE)
(72) Erfinder: Heismann, Björn, 91052, Erlangen (DE); Gross, Patrick, 91094, Langensendelbach (DE)

(56) Entgegenhaltungen:
- WO-A1-2004/024235
- WO-A1-2009/156896
- DE-A1-102008 007 245
- US-A1- 2009 088 625

## Beschreibung

Vorrichtung mit einer Kombination aus einer Magnetresonanzvorrichtung und einer Strahlentherapievorrichtung Die vorliegende Erfindung betrifft eine Vorrichtung mit einer Kombination aus einer Magnetresonanzvorrichtung, die zumindest einen Hauptmagneten zu einem Erzeugen eines Hauptmagnetfelds in einem Untersuchungsraum für eine Magnetresonanzmessung aufweist, und einer Strahlentherapievorrichtung, die zu einer Erzeugung eines Partikelstrahls vorgesehen ist und die eine Strahlführung aufweist, wobei die Magnetresonanzvorrichtung einen im Wesentlichen magnetfeldfreien Bereich aufweist und die Strahlführung des Partikelstrahls entlang des im Wesentlichen magnetfeldfreien Bereichs verläuft.

Der Hauptmagnet aufweist eine erste Magnetfeldspule zum Erzeugen eines ersten Magnetfelds und zumindest eine zweite Magnetfeldspule zum Erzeugen eines zweiten Magnetfelds, so dass der im Wesentlichen magnetfeldfreie Bereich innerhalb des Hauptmagneten vorliegt.

Im Rahmen einer Strahlentherapie soll im Allgemeinen ein Ziel innerhalb eines menschlichen Körpers bestrahlt werden, um beispielsweise eine Krankheit, insbesondere einen Tumor, zu behandeln. Dabei wird gezielt in einer Bestrahlungsregion (Isozentrum) des menschlichen Körpers von einer Strahlentherapievorrichtung eine hohe Strahlungsdosis eingebracht. Während einer Bestrahlung tritt häufig das Problem auf, dass die Bestrahlungsregion sich bewegt und/oder verschiebt. So verschiebt sich beispielsweise ein Tumor in einem Bauchbereich während eines Atemvorgangs eines Patienten. Andererseits kann ein Tumor innerhalb eines Zeitraums zwischen einer Bestrahlungsplanung und der tatsächlichen Bestrahlung sich vergrößert oder auch verkleinert haben.

Daher wurde vorgeschlagen, eine Lage eines Bestrahlungsziels in einem Patienten während der Bestrahlung durch eine medizinische Bildgebung zu kontrollieren. Dies ermöglicht, einen Strahl und/oder eine Strahlführung für die Bestrahlung zu steuern oder gegebenenfalls die Bestrahlung abzubrechen. Zudem ist eine Nachführung eines Strahlungsfokuses hinsichtlich einer tatsächlichen Lage der Bestrahlungsregion von hohem Interesse.

Insbesondere ist eine Kombination der Strahlentherapievorrichtung mit einer Magnetresonanzvorrichtung besonders vorteilhaft. Diese weist im Vergleich zu beispielsweise einer Computertomographievorrichtung eine hohe Weichteilauflösung auf, so dass in diesem Bereich ein vorteilhafter Kontrast dargestellt werden kann.

Für eine effiziente Bestrahlung wird eine Bestrahlungsquelle der Strahlentherapievorrichtung möglichst nah an einem Patienten positioniert. Hierzu ist zumeist die Bestrahlungsquelle zumindest teilweise innerhalb der Magnetresonanzvorrichtung und insbesondere innerhalb eines Magnetfelds der Magnetresonanzvorrichtung angeordnet. Jedoch weist diese Anordnung den Nachteil auf, dass eine Elektronenbahn von Elektronen eines Elektronenstrahls der Strahlentherapievorrichtung durch das Magnetfeld der Magnetresonanzvorrichtung gestört wird.

In WO 03/008986 A2 wird eine Teilung von Gradientenspulen der Magnetresonanzvorrichtung und ein angepasstes Design eines Hauptmagneten vorgeschlagen, so dass außerhalb der Magnetresonanzvorrichtung ein nahezu magnetfeldfreier Raum erzeugt wird. Jedoch weist diese Anordnung den Nachteil auf, dass die Vorrichtung eine große Ausdehnung aufweist und zudem nur ein Einstrahlwinkel für eine Strahlenbehandlung zur Verfügung steht. Zudem sind durch die geteilten Gradientenspulen deutliche Nachteile bei einer Bildqualität der medizinischen Bildgebung gegeben. Ferner muss eine Strahlung der Strahlentherapievorrichtung einen stählernen Körper des Magneten durchdringen, was zu einer Verschlechterung und/oder Degradierung des Strahlenprofils und der Strahlenintensität führt.

Aus der US 6,198,957 B1 ist ebenfalls eine Kombination einer Magnetresonanzvorrichtung mit einer Strahlentherapievorrichtung bekannt, bei der jedoch ein Röntgen- und/oder Gammastrahl zur Bestrahlung außerhalb der Magnetresonanzvorrichtung und damit außerhalb eines Wirkungsbereichs eines Magnetfelds erzeugt wird. Hierdurch wird der Röntgenstrahl in sehr großem Abstand zu dem eigentlichen Behandlungsbereich erzeugt, so dass die Vorrichtung ebenfalls eine große Ausdehnung, insbesondere bei einer Variation eines Einstrahlwinkels aufweist. Zudem muss aufgrund des großen Abstands eine hohe Strahlendosis erzeugt werden, um eine erforderliche Eindringtiefe der Strahlung für die Strahlenbehandlung zu erreichen.

Dokument WO 2004/024235 beschreibt eine Kombination aus einer Magnetresonanzvorrichtung und einer Strahlentherapievorrichtung. Der Hauptmagnet der Magnetresonanzvorrichtung weist innere Magnetspulen und äußere Magnetspulen, die derart zueinander angeordnet sind, dass außerhalb des Hauptmagneten ein im Wesentlichen magnetfeldfreier Bereich entsteht.

Zudem ist aus der DE 102008007245 A1 eine Führung eines Elektronenstrahls entlang einer Hauptachse der Magnetresonanzvorrichtung bekannt. Der Elektronenstrahl wird zu einer Kollision mit einem Traget um 90° umgelenkt. Ein Elektronenstrahl und das Target sind innerhalb einer Patientenaufnahme der Magnetresonanzvorrichtung angeordnet. Jedoch wird hierdurch ein für den Patienten zur Verfügung stehender Raum innerhalb der Magnetresonanzvorrichtung zusätzlich durch die Strahlentherapievorrichtung beschränkt.

Der vorliegenden Erfindung liegt insbesondere die Aufgabe zugrunde, eine Vorrichtung mit einer Kombination aus einer Magnetresonanzvorrichtung und einer Strahlentherapievorrichtung bereitzustellen, die einen kompakten Aufbau und eine qualitativ hochwertige Bildüberwachung durch eine Magnetresonanzmessung während einer Strahlentherapie zur Verfügung stellt. Die Aufgabe wird durch die Merkmale der unabhängigen Ansprüche gelöst. Weitere vorteilhafte Ausgestaltungen sind in den Unteransprüchen beschrieben.

Die Erfindung geht aus von einer Vorrichtung mit einer Kombination aus einer Magnetresonanzvorrichtung, die zumindest einen Hauptmagneten zu einem Erzeugen eines Hauptmagnetfelds in einem Untersuchungsraum für eine Magnetresonanzmessung aufweist, und einer Strahlentherapievorrichtung, die zu einer Erzeugung eines Partikelstrahls vorgesehen ist und die eine Strahlführung aufweist, wobei die Magnetresonanzvorrichtung (2) einen im Wesentlichen magnetfeldfreien Bereich aufweist und die Strahlführung des Partikelstrahls entlang des im Wesentlichen magnetfeldfreien Bereichs verläuft. Der Hauptmagnet aufweist eine erste Magnetfeldspule zum Erzeugen eines ersten Magnetfelds und zumindest eine zweite Magnetfeldspule zum Erzeugen eines zweiten Magnetfelds, so dass der im Wesentlichen magnetfeldfreie Bereich innerhalb des Hauptmagneten vorliegt.

Es wird vorgeschlagen, dass die Strahlführung des Partikelstrahls zumindest teilweise innerhalb des Hauptmagneten entlang des im Wesentlichen magnetfeldfreien Bereichs verläuft. In diesem Zusammenhang soll unter einem Hauptmagneten der Magnetresonanzvorrichtung insbesondere ein Magnet verstanden werden, der zu einem Anlegen und/oder Erzeugen eines konstanten und insbesondere homogenen Hauptmagnetfelds vorgesehen ist. Vorzugsweise ist der Hauptmagnet von einem supraleitenden Magneten gebildet. Zudem soll unter einem magnetfeldfreien Bereich insbesondere ein Bereich verstanden werden, in dem sich miteinander überlagernde Magnetfelder im Wesentlichen gegenseitig aufheben, so dass ein resultierendes Hauptmagnetfeld im Wesentlichen ausgelöscht ist. Der im Wesentlichen magnetfeldfreie Bereich kann dabei ein Restmagnetfeld aufweisen, wobei eine Stärke des Restmagnetfelds wesentlich kleiner ist als eine Stärke des Hauptmagnetfelds. Ferner soll unter einem Untersuchungsraum insbesondere ein Raum und/oder Bereich der Magnetresonanzvorrichtung verstanden werden, der zu einer Aufnahme eines Patienten vorgesehen ist und in dem eine bildgebende Untersuchung und/oder Messung an dem Patienten mittels der Magnetresonanzvorrichtung und eine Bestrahlung mittels der Strahlentherapievorrichtung vorgenommen wird. Unter einer Strahlentherapievorrichtung soll insbesondere eine Vorrichtung verstanden werden, die zu einem Bestrahlen eines Bereichs, beispielsweise eines Tumorbereichs eines Patienten, mit einer ionisierenden, hochenergetischen Strahlung vorgesehen ist, wobei die ionisierende, hochenergetische Strahlung von außen auf den Patienten einwirkt. Die ionisierende, hochenergetische Strahlung ist vorwiegend von einer Gammastrahlung und/oder einer Röntgenstrahlung und/oder einer Elektronenstrahlung gebildet, wobei eine Energie der Strahlung auf eine Gewebeart innerhalb des Behandlungsbereichs und/oder auf eine Position der Behandlungsbereichs innerhalb des Patienten und insbesondere unterhalb einer Haut des Patienten abgestimmt wird. Zudem ist auch eine Bestrahlung mit Neutronen und/oder Protonen und/oder schweren Ionen mittels der Strahlentherapievorrichtung denkbar. Ferner soll unter einem Partikelstrahl in diesem Zusammenhang insbesondere eine gerichtete Bewegung von einer Vielzahl an Partikeln und/oder Teilchen entlang einer einheitlichen Vorzugsrichtung verstanden werden, wie beispielsweise ein Strom von Partikeln und/oder Teilchen mit einer im Wesentlichen einheitlichen Flugrichtung. Die Strahlführung kann hierbei insbesondere einem vorgegebenen Strahlungsverlauf des Partikelstrahls gebildet sein.

Durch die erfindungsgemäße Vorrichtung kann vorteilhaft ein kompakter Aufbau der Vorrichtung erreicht werden, indem die Strahlentherapievorrichtung besonders platzsparend zumindest teilweise innerhalb der Magnetresonanzvorrichtung angeordnet werden kann. Des Weiteren ist auch eine unerwünschte Störung einer Bildgebungsmessung, die aufgrund einer Störung einer Homogenität des Hauptmagnetfelds durch den Partikelstrahls hervorgerufen werden könnte, reduziert und/oder verhindert, so dass eine qualitativ hochwertige Bildüberwachung durch eine Magnetresonanzmessung während einer Strahlentherapie erreicht werden kann. Darüber hinaus kann ein die Magnetresonanzvorrichtung mit einem Hochfeldmagnetfelds zum Einsatz kommen, da eine Beeinträchtigung von Magnetresonanzvorrichtung und Strahlentherapievorrichtung unterbunden ist. Es kann weiterhin auch eine unerwünschte Störung der Strahlführung durch das angelegte Hauptmagnetfeld aufgrund der Anordnung der Strahlführung in dem magnetfeldfreien Bereich unterdrückt werden.

Vorzugsweise verläuft die Strahlführung außerhalb eines Aufnahmebereichs der Magnetresonanzvorrichtung, wobei der Aufnahmebereich zu einer Aufnahme eines Patienten vorgesehen ist, so dass hierbei der Aufnahmebereich uneingeschränkt für den Patienten bereitgestellt werden, so dass Angstzustände, insbesondere aufgrund einer Klaustrophobie des Patienten innerhalb des Aufnahmebereichs, während einer Messung unterdrückt werden können.

Eine besonders kompakte Anordnung, bei der die Strahlentherapievorrichtung zumindest teilweise platzsparend in die Magnetresonanzvorrichtung integriert werden kann, kann erzielt werden, wenn die Strahlführung des Partikelstrahls zumindest teilweise innerhalb des Hauptmagneten entlang des im Wesentlichen magnetfeldfreien Bereichs verläuft. Für eine zumindest teilweise Integration der Strahlentherapievorrichtung innerhalb des Hauptmagneten eignet sich besonders vorteilhaft ein Hauptmagnet, der von einem als Minimum-Helium-Magnet ausgebildeten supraleitenden Magneten gebildet ist. Bei diesem Minimum-Helium-Magnet entfällt vorzugsweise eine Kühlung in einem Heliumbad. Vielmehr werden Windungen einer Magnetfeldspule des Hauptmagneten mit geringen Mengen Helium direkt gekühlt. Hierbei kann auf eine Einbettung des Minimum-Helium-Magneten in einem Gesamtkryostaten, wie dies bei supraleitenden Magneten einer Magnetresonanzvorrichtung üblich ist, verzichtet werden und der Minimum-Helium-Magnet in einem Vakuumgefäß, das wesentlich flexibler gestaltet werden kann und dabei unerwünschte Wärmebrücken verhindert, angeordnet werden. Innerhalb dieses Vakuumgefäßes kann beispielsweise die Strahlführung der Strahlentherapievorrichtung besonders platzsparend integriert werden.

Es wird ferner vorgeschlagen, dass der Hauptmagnet eine erste Magnetfeldspule zum Erzeugen eines ersten Magnetfelds und zumindest eine zweite Magnetfeldspule zum Erzeugen eines zweiten Magnetfelds aufweist, wobei der Strahlführung zumindest teilweise zwischen den beiden Magnetfeldspulen verläuft. Vorzugsweise ist das zweite Magnetfeld von einem dem ersten Magnetfeld zumindest teilweise entgegenwirkenden Gegenmagnetfeld gebildet, so dass eine zumindest teilweise Auslöschung und/oder Aufheben der beiden Magnetfelder, insbesondere in einem Raum zwischen den beiden Magnetspulen, erreicht werden kann und damit ein im wesentlicher magnetfeldfreier Bereich und/oder Raum innerhalb der Hauptmagneten. Vorzugsweise weist die erste Magnetfeldspule einen Durchmesser auf, der größer ist als ein Durchmesser der zumindest zweiten Magnetfeldspule, so dass die zumindest eine zweite Magnetfeldspule platzsparend innerhalb eines von Windungen der ersten Magnetfeldspule umschlossenen Bereichs angeordnet werden kann. Der magnetfeldfreie Bereich zwischen den beiden Magnetfeldspulen erstreckt sich vorzugsweise im Wesentlichen entlang und/oder parallel einer Länge der beiden Magnetfeldspulen, so dass die Strahlführung im Wesentlichen parallel zur Länge zwischen den beiden Magnetfeldspulen verläuft.

Weiterhin wird vorgeschlagen, dass die Strahlführung zumindest teilweise parallel zu einer Richtung und/oder einer Orientierung einer magnetischen Flussdichte des Hauptmagnetfelds verläuft. Es kann hierdurch eine im Wesentlichen magnetkraftfreie Strahlführung des Partikelstrahls innerhalb des Hauptmagneten erreicht werden.

Es wird ferner vorgeschlagen, dass der Hauptmagnet zumindest zwei einzelne Magnete aufweist, die zusammen in einem Vakuumgefäß angeordnet sind. Es kann eine vorteilhafte Kühlung unter Einsparung von großen Mengen Helium erreicht werden und dabei besonders platzsparend die Strahlführung innerhalb des Hauptmagneten angeordnet werden.

In einer vorteilhaften Weiterbildung der Erfindung wird vorgeschlagen, dass die Strahlführung entlang einer zumindest teilweise gekrümmten Trajektorie innerhalb des Hauptmagneten verläuft. Es kann hierbei ein vorhandenes Restmagnetfeld innerhalb des Hauptmagneten entlang der Strahlführung vorteilhaft durch einen Krümmungsradius der Trajektorie kompensiert werden. Dabei ist eine aktive und/oder passive Korrektur einer Bahn des Partikelstrahls denkbar, beispielsweise aufgrund einer aktiven Strahlablenkung durch lokale Magnetspulen entlang der Strahlführung usw.

Zudem wird vorgeschlagen, dass die Strahlentherapievorrichtung eine Beschleunigereinheit aufweist, die zumindest teilweise innerhalb der Magnetresonanzvorrichtung angeordnet ist, wobei die Beschleunigereinheit vorteilhafterweise von einer Linearbeschleunigereinheit gebildet ist. Es kann hierbei eine besonders kompakte Anordnung der Beschleunigereinheit innerhalb der Vorrichtung erreicht werden. Besonders vorteilhaft kann dies erreicht werden, wenn die Linearbeschleunigereinheit zumindest teilweise innerhalb der Hauptmagneten angeordnet ist.

In diesem Zusammenhang soll unter einer Linearbeschleunigereinheit (Linac) insbesondere eine Einheit zu einem Beschleunigen von elektrisch geladenen Teilchen und/oder Partikeln, insbesondere Elektronen, verstanden werden, wobei die Teilchen und/oder Partikel auf einer geraden Bahn beschleunigt werden. Eine Beschleunigung des Partikelstrahls erfolgt hierbei durch elektrische Wechselfelder in einem zylinderförmigen Hohlleiter. Der Hohlleiter ist hierbei vorzugsweise in einer Vakuumröhre angeordnet, so dass unerwünschte Kollisionen der Strahlpartikel mit Luftmolekülen und/oder Luftpartikeln verhindert sind. Für eine Strahlentherapie können Elektronen beispielsweise auf Energien bis auf eine Größenordnung von mehreren MeV beschleunigt werden. Zudem ist es auch möglich, dass die Strahlentherapievorrichtung eine zur Linearbeschleunigereinheit alternative Beschleunigereinheit aufweist.

Zudem kann die Beschleunigereinheit auch ein zu einem Vakuumgefäß des Hauptmagneten separates Vakuumgefäß aufweisen. Hierdurch kann bei einem Eintritt und/oder einem Austritt des Partikelstrahls in den Hauptmagneten beispielsweise eine unerwünschte Absorption des Partikelstrahls, insbesondere eine Betaabsorption von Elektronen des Partikelstrahls, an dem Vakuumgefäß des Hauptmagneten verhindert werden.

Darüber hinaus wird vorgeschlagen, dass die Beschleunigereinheit zumindest teilweise innerhalb eines Vakuumgefäßes des Hauptmagneten angeordnet ist. Es können hierbei weitere Bauteile und damit insbesondere Kosten für eine separates Vakuumgefäß und/oder eine separate Vakuumeinheit der Strahlentherapievorrichtung eingespart werden.

Zudem wird vorgeschlagen, dass der Hautmagnet ein Vakuumgefäß mit zumindest einem Eintritts- und/oder Austrittsfenster aufweist, durch das die Partikel des Partikelstrahls in das Vakuumgefäß gelangen können und/oder das Vakuumgefäß wieder verlassen können. Vorzugsweise ist das Eintritts- und/oder das Austrittsfenster aus einem Material gebildet, das eine geringe Wechselwirkung mit dem Partikelstrahl aufweist, so dass bei einem Auftreffen des Partikelstrahls auf das Eintritts- und/oder das Austrittsfenster Verluste in der Strahlintensität und/oder Abweichung in der Strahlführung und/oder eine Strahlaufweitung reduziert und/oder verhindert werden können. Es kann eine unerwünschte Strahlablenkung und/oder Streuung von Partikeln des Partikelstrahls bei einem Eintritt und/oder Austritt des Partikelstrahls in das Vakuumgefäß zumindest reduziert oder verhindert werden.

In einer weiteren Ausgestaltung der Erfindung wird vorgeschlagen, dass die Strahlentherapievorrichtung zumindest ein Targetelement aufweist, das innerhalb eines Vakuumgefäßes der Strahlentherapievorrichtung und/oder des Hauptmagneten angeordnet ist, wodurch eine insbesondere platzsparende Anordnung und/oder Positionierung des Targets innerhalb der Magnetresonanzvorrichtung erreicht werden kann. Zudem kann eine platzsparende Anordnung in einer Nähe zu einem Patienten und damit besonders nah an einer Behandlungsregion erreicht werden. An dem Targetelement werden die auf das Targetelement auftreffenden Elektronen des Elektronenstrahls des Linearbeschleunigers abgebremst und senden dabei eine Bremsstrahlung aus, die von den hochenergetischen Photonen gebildet ist. Das Targetelement kann hierbei von einem Transmissionstargetelement oder einem Reflexionstargetelement gebildet sein.

Eine vorteilhafte Bestrahlung eines Behandlungsbereichs aus unterschiedlichen Bestrahlungswinkeln kann erreicht werden, wenn die Strahlentherapievorrichtung zumindest ein Targetelement aufweist, das von einem um einen Aufnahmebereich der Magnetresonanzvorrichtung umlaufenden Targetring gebildet ist. Vorzugsweise weist hierzu die Strahlentherapievorrichtung zumindest zwei oder mehr Strahlführungen auf, die innerhalb der Magnetresonanzvorrichtung und insbesondere innerhalb des Hauptmagneten angeordnet sind und die je nach Wahl eines Bestrahlungswinkels eingesetzt werden können. Zudem kann die Strahlentherapievorrichtung einen Strahlführung vorsehen, die innerhalb des Hauptmagneten im Wesentlichen eine Form eines Zylindermantelfläche aufweist, mittels der ein Beschuss des Targetrings und/oder ein Auftreffen des Partikelstrahls auf den Targetring über einen ausgewählten Teilwinkelbereich ermöglicht wird.

Ferner wird vorgeschlagen, dass die Strahlentherapievorrichtung zumindest ein Kollimatorelement aufweist, das innerhalb eines Vakuumgefäßes der Strahlentherapievorrichtung und/oder des Hauptmagneten angeordnet ist. Es kann eine insbesondere platzsparende Anordnung und/oder Positionierung des Kollimatorelements innerhalb der Magnetresonanzvorrichtung und zudem eine platzsparende Anordnung in einer Nähe zu der Behandlungsregion und damit besonders nah am Patienten erreicht werden. Das Kollimatorelement ist insbesondere zu einer parallelen Ausrichtung einer Gamma- und/oder Röntgenstrahlung eines Gamma- und/oder Röntgenstrahls vorgesehen. Vorzugsweise werden hierbei Photonen mit einer von der parallelen Strahlungsrichtung abweichenden Orientierung aus dem Photonenstrahl herausfiltert.

Es wird des Weiteren vorgeschlagen, dass die Strahlentherapievorrichtung zumindest ein Kollimatorelement aufweist, das um zumindest eine Achse und/oder entlang zumindest einer Achse bewegbar angeordnet ist. Hierdurch kann ein Gamma- und/oder Röntgenstrahl auf ein gewünschtes Strahlprofil und/oder auf einen gewünschten Behandlungsbereich für eine Strahlentherapie gebracht werden. Das zumindest eine Kollimatorelement ist hierzu besonders vorteilhaft um eine Achse kippbar und/oder drehbar ausgeführt.

Eine besonders vorteilhafte Bestrahlung, insbesondere aus unterschiedlichen Bestrahlungspositionen, eines Bestrahlungsbereichs in einem Patienten kann erreicht werden, wenn die Strahlentherapievorrichtung zumindest zwei Strahlführungen für den Partikelstrahl aufweist und die zumindest zwei Strahlführungen innerhalb der Magnetresonanzvorrichtung angeordnet sind. Es kann hierbei eine Auswahl einer Strahlführung anhand einer gewünschten Einstrahlposition gewählt werden. Zudem können die zumindest zwei Strahlführungen für ein zeitgleiches Einstrahlen aus unterschiedlichen Bestrahlungswinkeln verwendet werden. Eine besonders kompakte Anordnung kann erreicht werden, wenn die zumindest zwei Strahlführungen für den Partikelstrahl zumindest teilweise innerhalb des Hauptmagneten angeordnet sind.

Ferner wird vorgeschlagen, dass die Strahlführung zusammen mit der zumindest teilweisen Magnetresonanzvorrichtung entlang wenigstens einer Richtung verschiebbar angeordnet ist. Es kann eine vorteilhafte Positionierung des Partikelstrahls und/oder des Gamma- und/oder Röntgenstrahls bezüglich des Behandlungsbereichs erreicht werden. Zudem kann eine kompakte Ausführung der Vorrichtung beibehalten werden und/oder ein hohes Hauptmagnetfeld für eine Magnetresonanzmessung während einer Bestrahlung aus unterschiedlichen Bestrahlungspositionen aufrechterhalten werden. Besonders vorteilhaft ist zudem die Strahlführung zumindest teilweise zusammen mit der Magnetresonanzvorrichtung entlang zwei oder drei Raumrichtungen verschiebbar angeordnet, wobei die Raumrichtungen vorzugsweise orthogonal zueinander ausgerichtet sind.

Des Weiteren wird vorgeschlagen, dass die Strahlführung zusammen mit der zumindest teilweisen Magnetresonanzvorrichtung um zumindest eine Achse drehbar angeordnet ist. Vorzugsweise verläuft die Achse durch den Aufnahmebereich für den Patienten, so dass eine effiziente Bestrahlung des Patienten, insbesondere des Behandlungsbereichs des Patienten, von unterschiedlichen Winkelpositionen erreicht werden kann.

Besonders vorteilhaft weist die Magnetresonanzvorrichtung eine Patientenliege zu einem Einführen eines Patienten in einen Aufnahmebereich auf, wobei die Patientenliege entlang zumindest zwei Richtungen verschiebbar angeordnet ist. Es kann eine effektive Positionierung des Patienten hinsichtlich einer Ausrichtung und/oder Orientierung des Partikelstrahls und/oder des Gamma und/oder Röntgenstrahls der Strahlentherapievorrichtung erreicht werden.

Zudem wird vorgeschlagen, dass der Partikelstrahl und ein aus dem Partikelstrahl erzeugter Gamma- und/oder Röntgenstrahl im Wesentlichen orthogonal zueinander ausgerichtet sind. Es kann hierbei eine vorteilhafte Einstrahlposition eines Behandlungsstrahls hinsichtlich einer Position des Patienten und insbesondere hinsichtlich eines Behandlungsbereichs innerhalb des Patienten erreicht werden. Insbesondere kann hierbei der Behandlungsstrahl im Wesentlichen senkrecht zu einer Kopf-Fuß-Achse des Patienten auf den Patienten gerichtet werden.

Besonders vorteilhaft ist die Magnetresonanzvorrichtung von einer Hochfeldmagnetresonanzvorrichtung gebildet, so dass eine hohe Qualität in den Signalen der aufgenommenen Magnetresonanzmessungen erreicht werden kann. Vorzugsweise weist das Magnetfeld hierbei eine Magnetfeldstärke von mindestens 3 Tesla und vorteilhafterweise von mindestens 5 Tesla auf.

Weitere Vorteile ergeben sich aus der folgenden Zeichnungsbeschreibung. In der Zeichnung sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln Betrachten und zu sinnvollen weiteren Kombinationen zusammenfassen.

Es zeigen:
- FIG 1: eine erfindungsgemäße Vorrichtung in einer schematischen Darstellung,
- FIG 2: eine Detailansicht in der Vorrichtung aus FIG 1 in einer schematischen Darstellung,
- FIG 3: eine alternative Ausgestaltung der Vorrichtung in einer schematischen Darstellung,
- FIG 4: eine alternative Ausgestaltung der Vorrichtung mit mehreren Strahlführungen in einer schematischen Darstellung und
- FIG 5: eine alternative Ausgestaltung der Vorrichtung mit einem Targetring in einer schematischen Darstellung.

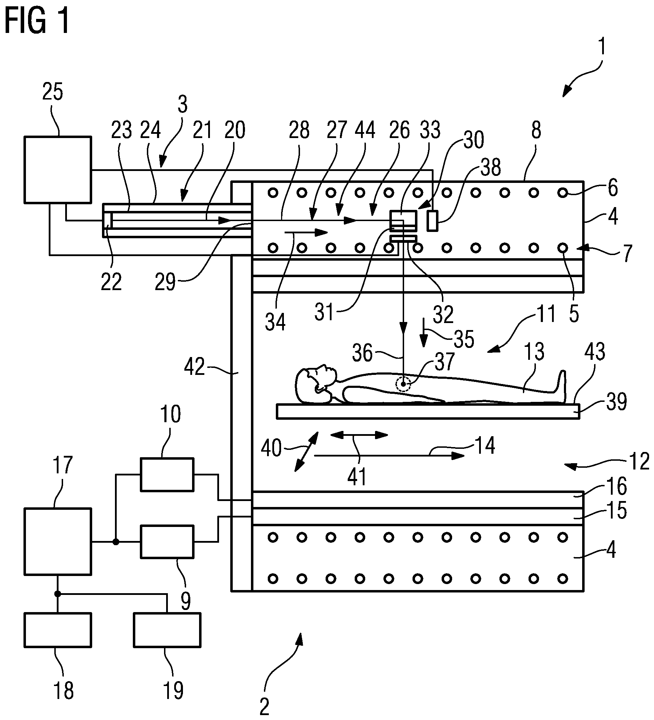

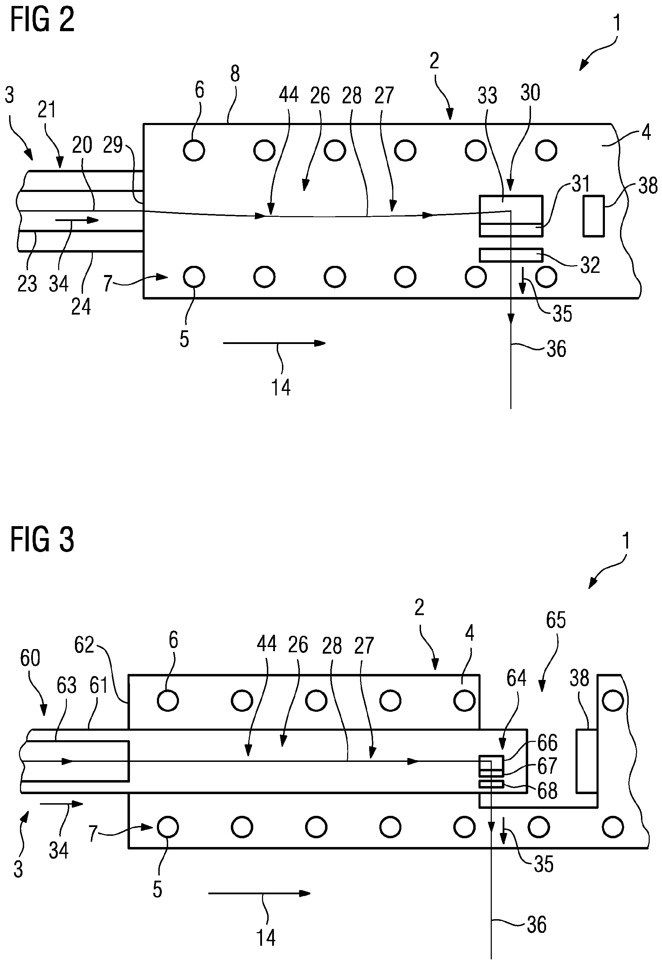

In FIG 1 und 2 ist eine Vorrichtung 1 mit einer Kombination aus einer Magnetresonanzvorrichtung 2 und einer Strahlentherapievorrichtung 3 dargestellt. Die Magnetresonanzvorrichtung 2 ist von einer Hochfeldmagnetresonanzvorrichtung gebildet und umfasst einen Hauptmagneten 4, der im Betrieb der Magnetresonanzvorrichtung 2 zu einem Erzeugen eines insbesondere homogenen und konstanten Hauptmagnetfelds vorgesehen ist. Der Hauptmagnet 4 ist hierzu von einem Hochfeldmagnetresonanzmagneten gebildet und umfasst zumindest zwei Magnetfeldspulen 5, 6, die insbesondere von supraleitenden Magnetfeldspulen 5, 6 gebildet sind. Eine der beiden Magnetfeldspulen 5 ist von einer Primärspule gebildet, die mehrere Wicklungsebenen 7 aufweist und die zu einer Erzeugung des homogenen Magnetfelds vorgesehen ist (FIG 2). Die weitere Magnetfeldspule 6 ist von einer Streufeldgegenspule gebildet. Die Primärspule weist einen Wicklungsquerschnitt auf, der kleiner ist als ein Wicklungsquerschnitt der Streufeldgegenspule, so dass die Primärspule innerhalb der Streufeldgegenspule angeordnet ist.

Der Hauptmagnet 4 ist im vorliegenden Beispiel von einem Minimum-Helium-Magneten gebildet. Dieser umfasst ein einziges Vakuumgefäß 8, in dem die beiden Magnetfeldspulen angeordnet 5, 6 sind. Anstatt einer Kühlung der Magnetfeldspulen 5, 6 in einem Heliumbad befindet sich innerhalb des Vakuumgefäßes 8 eine geringe Menge von Helium, die für eine direkte Kühlung von Windungen der beiden Magnetfeldspulen 5, 6 vorgesehen ist.

Das von dem Hauptmagneten 4 erzeugte Hauptmagnetfeld wirkt in einem Untersuchungsraum 11 der Magnetresonanzvorrichtung 2, wobei der Untersuchungsraum 11 im Wesentlichen von einem Aufnahmebereich 12 zur Aufnahme eines Patienten 13 für eine Magnetresonanzmessung gebildet ist (FIG 1). Eine Orientierung und/oder eine Richtung einer magnetischen Flussdichte 14 des Hauptmagnetfelds verläuft dabei im Wesentlichen senkrecht zu einem Wicklungsquerschnitt der beiden Magnetfeldspulen 5, 6. Zudem ist das homogene Hauptmagnetfeld im Wesentlichen auf den von den Magnetfeldspulen 5, 6 umschlossenen Aufnahmebereich 12 konzentriert.

Des Weiteren umfasst die Magnetresonanzvorrichtung 2 eine Gradienteneinheit 15 zur Erzeugung von Magnetfeldgradienten (FIG 1). Die Gradienteneinheit 15 weist nicht näher dargestellte Gradientenspulen auf, die magnetische Gradientenfelder zur selektiven Schichtanregung und/oder zur Ortskodierung von Magnetresonanzsignalen entlang von drei Raumrichtungen einstrahlen. Zur Anregung einer Polarisation, die sich in dem von dem Hauptmagneten 4 erzeugten Hauptmagnetfeld der Magnetresonanzvorrichtung 2 einstellt, ist eine Hochfrequenzspuleneinheit 16 vorgesehen. Diese strahlt ein Hochfrequenzfeld, beispielsweise in Form eines HF-Pulses, in den Patienten 13 ein, um eine Magnetisierung aus einer Gleichgewichtslage auszulenken. Mittels der Hochfrequenzspuleneinheit 16 können in einem Messbetrieb Magnetresonanzsignale aus dem Untersuchungsraum 11 aufgenommen werden. Des Weiteren umfasst die Magnetresonanzvorrichtung 2 einen Gradientenverstärker 9 zu einer Steuerung der Gradientenspulen und einen Hochfrequenzverstärker 10 zu einer Steuerung von Hochfrequenzspulen. Zu einer Steuerung des Gradientenverstärkers 9 und des Hochfrequenzverstärkers 10 umfasst die Magnetresonanzvorrichtung 2 eine Steuereinheit 17. Die Steuereinheit 17 steuert zentral die Magnetresonanzvorrichtung 2, wie beispielsweise das selbsttätige Durchführen einer ausgewählten bildgebenden Gradientenechosequenz. Die Steuereinheit 17 steuert zentral die Magnetresonanzvorrichtung 2, wie beispielsweise das selbsttätige Durchführen einer ausgewählten bildgebenden Gradientenechosequenz. Zu einer Eingabe von Magnetresonanzparametern umfasst die Magnetresonanzvorrichtung 2 eine Eingabeeinheit 18. Des Weiteren umfasst die Magnetresonanzvorrichtung 2 eine Anzeigeeinheit 19, mittels der beispielsweise Magnetresonanzbilder dargestellt werden können.

Die Strahlentherapievorrichtung 3 ist im Betrieb zu einer Erzeugung eines Partikelstrahls 20 vorgesehen. Hierzu weist die Strahlentherapievorrichtung 3 eine Beschleunigereinheit auf, der von einer Linearbeschleunigereinheit 21 gebildet ist. Die Linearbeschleunigereinheit 21 ist als Elektronenbeschleunigereinheit ausgebildet. Grundsätzlich ist eine zu der Linearbeschleunigereinheit 21 und/oder der Elektronenbeschleunigereinheit alternative Ausgestaltung der Beschleunigereinheit denkbar, wie beispielsweise eine Protonenbeschleunigereinheit etc.

Die Linearbeschleunigereinheit 21 umfasst eine Elektronenquelle 22, wie beispielsweise eine Wolframkathode, die freie Elektronen erzeugt. Diese Elektronen werden anschließend beschleunigt und der von einem Elektronenstrahl gebildeter Partikelstrahl 20 erzeugt. Hierzu weist die Linearbeschleunigereinheit 21 einen Hohlraumresonator 23 auf. In dem Hohlraumresonator 23 werden elektrische Felder von stehenden elektromagnetischen Wellen erzeugt. Innerhalb des Hohlraumresonators 23 sind mehrere Zellen nacheinander angeordnet, wobei eine Länge der einzelnen Zellen derart gewählt ist, dass sich das elektrischen Feld der stehenden Welle einer Zelle umkehrt, sobald ein Elektron in die darauffolgende Zelle eintritt. Es wird somit eine kontinuierliche Beschleunigung der Elektronen auf eine Energie von einigen MeV gewährleistet. Des Weiteren umfasst die Linearbeschleunigereinheit 21 ein von einer Vakuumröhre gebildetes Vakuumgefäß 24, innerhalb dessen der Hohlraumresonator 23 angeordnet ist, so dass in den einzelnen Zellen des Hohlraumresonators 23 ein Vakuum anliegt. Für eine Steuerung und/oder Regelung der Linearbeschleunigereinheit 21 weist die Strahlentherapievorrichtung 2 eine Steuereinheit 25 auf.

Eine Strahlentherapiebehandlung mittels der Strahlentherapievorrichtung 3 erfolgt gleichzeitig zu einer Magnetresonanzmessung mittels der Magnetresonanzvorrichtung 2, so dass die Strahlentherapiebehandlung effektiv an beispielsweise eine Bewegung des Patienten angepasst werden kann. Die Strahlentherapievorrichtung 2 ist hierzu zumindest teilweise innerhalb der Magnetresonanzvorrichtung 3 integriert. Die Strahlentherapievorrichtung 2 weist eine Strahlführung 44 für den Elektronenstrahl auf, die hierbei durch den Hauptmagneten 4 der Magnetresonanzvorrichtung 2 verläuft, wobei die Strahlführung 44 des Elektronenstrahls zwischen einer Wicklungsebene der Primärspule und der Streufeldgegenspule verläuft. In der Streufeldgegenspule wird im Betrieb der Magnetresonanzvorrichtung 2 ein zu der Primärspule entgegengerichtetes Magnetfeld erzeugt, so dass zwischen der Primärspule und der Streufeldgegenspule ein Niedermagnetfeldbereich 26 entsteht. Dieser Niedermagnetfeldbereich 26 ist zumindest teilweise von einem im Wesentlichen magnetfeldfreien Bereich 27 gebildet, wobei die Strahlführung des Elektronenstrahls entlang des im Wesentlichen magnetfeldfreien Bereichs 27 verläuft. Die Strahlführung des Elektronenstrahls verläuft somit außerhalb des Aufnahmebereichs 12 der Magnetresonanzvorrichtung 2 zur Aufnahme des Patienten 13. Der magnetfeldfreie Bereich 27 ist im Wesentlichen von einer welligen Fläche, die sich zwischen den beiden Magnetfeldspulen 5, 6 des Hauptmagneten 4 zylinderförmig erstreckt, gebildet.

Die Strahlführung verläuft hierbei im Wesentlichen parallel zur magnetischen Flussdichte 14 des Hauptmagnetfelds, wobei die Strahlführung des Elektronenstrahls entlang einer zumindest teilweise gebogenen Trajektorie 28 verläuft. Die zumindest teilweise gebogene Trajektorie 28 dient dazu, ein Restmagnetfeld entlang der Strahlführung zu kompensieren. Zudem verläuft die Strahlführung und/oder die Trajektorie 28 im Wesentlichen parallel zur magnetischen Flussdichte 14 des Hauptmagnetfelds des Hauptmagneten 4. Die Strahlführung kann hierbei entlang der Trajektorie 28 aktiv umgelenkt werden, beispielsweise mittels Lokalspulen usw. Zudem kann auch ein passives Umlenken vorgesehen sein, bei dem ein Restmagnetfeld zwischen den beiden Spulen entlang der Trajektorie 28 für die Unlenkung ausgenutzt wird. Hierbei kann beispielsweise aufgrund des angelegten Magnetfelds in den einzelnen Magnetfeldspulen 5, 6 ein eventuell vorhandenes Restmagnetfeld entlang des gewünschten Strahlverlaufs berechnet werden. Der Partikelstrahl 20 kann dann anschießend mit einem entsprechenden Einstrahlwinkel in das Restmagnetfeld des Hauptmagneten 4 eingestrahlt werden, wobei das Restmagnetfeld eine Ablenkung bewirkt, so dass der Partikelstrahl 20 an definierter Position auf eine Targeteinheit 30 trifft.

Die Linearbeschleunigereinheit 21 der Strahlentherapievorrichtung 2 ist außerhalb des Hauptmagneten 4 der Magnetresonanzvorrichtung 2 angeordnet. Für einen Eintritt des Elektronenstrahls in den Hauptmagneten 4 und insbesondere in dessen Vakuumgefäß 8 weist dieses ein Eintrittsfenster 29 auf, wobei das Vakuumgefäß 24 der Linearbeschleunigereinheit 21 direkt an das Eintrittsfenster 29 anschließt. Um eine Wechselwirkung, insbesondere eine Ablenkung und/oder eine Streuung der Elektronen des Elektronenstrahls mit dem Eintrittsfenster 29 des Vakuumgefäßes 8 zu verhindern, ist das Eintrittsfenster 29 aus einem Material gebildet, das für die Elektronen des Elektronenstrahls im Wesentlichen transparent ist.

Des Weiteren umfasst die Strahlentherapievorrichtung 2 eine Targeteinheit 30, die ein Targetelement 31, ein Kollimatorelement 32 und eine Strahlumlenkungseinheit 33 umfasst. Die Targeteinheit 30 ist entlang einer Richtung 34 einer Geschwindigkeit der beschleunigten Elektronen des Elektronenstrahls nach dem Hohlraumresonator 23 der Linearbeschleunigereinheit 21 angeordnet. Zudem ist die Targeteinheit 30 innerhalb des Vakuumgefäßes 8 des Hauptmagneten 4 angeordnet, so dass eine unerwünschte Strahlablenkung aufgrund einer Kollision von beschleunigten Elektronen des Elektronenstrahls mit Luftmolekülen und/oder Luftpartikeln verhindert ist. Nachdem die beschleunigten Elektronen des Elektronenstrahls den Hohlraumresonator 23 und einen Teilbereich des Hauptmagneten 4 durchlaufen haben, treffen sie zunächst auf die Strahlumlenkungseinheit 33. Hier wird der Elektronenstrahl um ca. 90° auf das Targetelement 31 umgelenkt, wobei eine Richtung 35 der Geschwindigkeit der Elektronen auf den Aufnahmebereich 12 gerichtet ist. Um eine weitere Ablenkung der Elektronen zu verhindern, die diese aufgrund der im Wesentlichen senkrechten Ausrichtung der Richtung 35 der Geschwindigkeit der Elektronen zu der Richtung der magnetischen Flussdichte 14 des Hauptmagnetfelds erfahren, ist direkt nach der Strahlumlenkungseinheit 33 das Targetelement 31 angeordnet. Das Targetelement 31 ist von einem Transmissionstargetelement, beispielsweise aus einem Wolframblech, gebildet. An dem Targetelement 31 werden die beschleunigten Elektronen abgebremst, wobei hierbei eine Gammastrahlung und/oder Röntgenstrahlung erzeugt wird. An dem Transmissionstargetelement wird ein Gamma- und/oder Röntgenstrahl 36 erzeugt, der einen Winkel zu dem an dem Transmissionstargetelement 31 einfallenden Elektronenstrahlen von im Wesentlichen 0° aufweist. Aufgrund der Anordnung der Strahlumlenkungseinheit 33 und des Targetelements 31 innerhalb des Hauptmagneten 4 sind diese magnetresonanzkompatibel ausgebildet und aus einen nichtmagnetisierbaren Material gebildet.

Entlang der Richtung 35 des Elektronenstrahls ist nach dem Targetelement 21 das Kollimatorelement 32 angeordnet. Mittels des Kollimatorelements 32 wird aus der diffusen Gamma- und/oder Röntgenstrahlung eine parallele Gamma- und/oder Röntgenstrahlung erzeugt und diese auf einen Behandlungsbereich 38 des Patienten 13 fokussiert. Hierbei wird ein paralleler Strahlenverlauf der Gamma- und/oder Röntgenstrahlung mit einem kleinen Bestrahlungsfokus für eine Strahlentherapie zur Verfügung gestellt. Das Kollimatorelement 32 ist zudem um eine Achse bewegbar angeordnet, so dass der Bestrahlungsfokus auf den Behandlungsbereich 37, beispielsweise ein Tumorgewebe, eingestellt werden kann. Eine Steuerung und/oder eine Einstellung des Kollimatorelements 32 erfolgt mittels der Steuereinheit 25, wobei hierzu Steuerparameter und/oder Einstellungsparameter von einem Bediener, beispielsweise einem Arzt, über eine nicht näher dargestellte Eingabeeinheit eingegeben werden können. Das Kollimatorelement 32 ist ebenfalls innerhalb des Hauptmagneten 4 angeordnet und hierzu magnetresonanzkompatibel ausgebildet und aus einem nichtmagnetisierbaren Material gebildet.

Des Weiteren umfasst die Strahlentherapievorrichtung 3 eine Kontrolleinheit 38. Mittels der Kontrolleinheit 38 wird eine Strahlqualität des an dem Targetelement 31 erzeugten Gamma- und/oder Röntgenstrahls 36, beispielsweise hinsichtlich einer Strahlungsdosis und/oder eines Strahlungsfokuses, überwacht. Eine Steuerung der Kontrolleinheit 38 erfolgt durch die Steuereinheit 25 der Strahlentherapievorrichtung 3. Sobald Parameter des Gamma- und/oder Röntgenstrahls 36 von einem vorbestimmten Wert abweichen, wird von Steuereinheit 25 daraufhin die Strahlenbehandlung beendet. Die Kontrolleinheit 38 ist hierzu in einer Nähe zu dem Targetelement 31 innerhalb des Vakuumgefäßes 8 des Hauptmagneten 4 und entlang des Strahlverlaufs nach dem Targetelement 31 angeordnet. Zudem ist die Kontrolleinheit 38 ebenfalls magnetresonanzkompatibel ausgebildet und aus einem nichtmagnetisierbaren Material gebildet.

Ferner ist die Vorrichtung 1 dazu vorgesehen, eine Nachführung eines Bestrahlungsfokus des Gamma- und/oder Röntgenstrahls 36 vorzunehmen. Eine Nachführung kann erforderlich sein, wenn beispielsweise das Isozentrum und/oder der Behandlungsbereich 38 der Strahlenbehandlung sich während der Strahlenbehandlung bewegt, beispielsweise aufgrund einer Atmung und/oder einer Bewegung des Patienten 13. Des Weiteren kann das Isozentrum und/oder der Behandlungsbereich 38 eine größere Ausdehnung aufweisen als eine Ausdehnung des Bestrahlungsfokus des Gamma- und/oder Röntgenstrahls 36, so dass für eine vollständige Bestrahlung, beispielsweise eines Tumorgewebes, eine Nachführung des Gamma- und/oder Röntgenstrahls 36 erforderlich ist. Die Nachführung erfolgt anhand von Magnetresonanzaufnahmen, die den Behandlungsbereich lokalisieren. Für die Nachführung des Bestrahlungsfokus des Gamma- und/oder Röntgenstrahls 36 hinsichtlich einer Bewegung und/oder Ausdehnung des Isozentrums sieht die Vorrichtung 1 zwei Möglichkeiten vor. Zum einen weist die Magnetresonanzvorrichtung 2 eine Patientenliege 39 auf, die zu einem Einbringen des Patienten 13 in den Aufnahmebereich 12 der Magnetresonanzvorrichtung 2 vorgesehen ist. Die Patientenliege 39 ist magnetresonanzkompatibel ausgebildet und aus einem nichtmagnetisierbaren Material gebildet. Zudem ist die Patientenliege 39 entlang zwei Raumrichtungen innerhalb des Aufnahmebereichs 12 bewegbar ausgebildet. Die zwei Raumrichtungen sind von einer x-Richtung 40 und einer z-Richtung 41 gebildet und jeweils orthogonal zueinander ausgerichtet. Die z-Richtung 41 ist zudem entlang einer Richtung eines Einschubvorgangs zu einem Einschieben der Patientenliege 39 in den Aufnahmebereich 12 ausgerichtet. Zusätzlich kann die Patientenliege 41 auch entlang einer dritten Raumrichtung, die von einer y-Richtung gebildet ist, bewegbar angeordnet sein, wobei die dritte Raumrichtung orthogonal zu der ersten und der zweiten Raumrichtung ausgerichtet ist.

Eine weitere Möglichkeit der Nachführung des Gamma- und/oder Röntgenstrahls 36 hinsichtlich des Isozentrums und/oder des Behandlungsbereichs 37 besteht darin, das zumindest die Linearbeschleunigereinheit 21 und die Targeteinheit 30 der Strahlentherapievorrichtung 3 zusammen mit zumindest dem Hauptmagneten 4 der Magnetresonanzvorrichtung 2 bewegbar angeordnet ist. Die Bewegung der Linearbeschleunigereinheit 21 und der Targeteinheit 30 der Strahlentherapievorrichtung 3 muss hierbei zusammen mit zumindest dem Hauptmagneten 4 der Magnetresonanzvorrichtung 2 erfolgen, so dass die Strahlführung des Elektronenstrahls stets im nahezu feldfreien Bereich 27 des Hauptmagneten 4 angeordnet sein kann. Hierzu weist die Vorrichtung 1 eine Positioniereinheit 42 auf, die die Linearbeschleunigereinheit 21 und die Targeteinheit 30 der Strahlenvorrichtung 3 zusammen mit zumindest den Hauptmagneten 4 der Magnetresonanzvorrichtung 2 entlang der drei Raumrichtungen bezüglich des Patienten 13 und/oder der Patientenliege 39 bewegt. Alternativ hierzu kann zudem die Bewegung entlang nur einer oder zwei Raumrichtungen möglich sein.

Zudem kann es zudem vorgesehen sein, dass zusätzlich weitere Baueinheiten und/oder Bauelemente der Magnetresonanzvorrichtung 2 zusammen mit der Linearbeschleunigereinheit 21 und der Targeteinheit 30 entlang der drei Raumrichtungen zu einer Nachführung des Gamma- und/oder Röntgenstrahls von der Positioniereinheit 42 bewegt werden. Darüber hinaus können auch weitere Baueinheiten und/oder weitere Bauelemente der Strahlentherapievorrichtung 3 für die Nachführung des Gamma- und/oder Röntgenstahls entlang der drei Raumrichtungen von der Positioniereinheit 42 bewegt werden.

Des Weiteren sieht die Vorrichtung 1 eine Bestrahlung des Behandlungsbereichs 37 aus unterschiedlichen Bestrahlungswinkeln und/oder aus unterschiedlichen Bestrahlungspositionen vor. Der Bestrahlungswinkel ist dabei von einem dreidimensionalen Raumwinkel bezüglich einer Liegefläche 43 der Patientenliege 39 gebildet. Hierzu werden die Linearbeschleunigereinheit 21 und die Targeteinheit 30 der Strahlentherapievorrichtung 3 zusammen mit zumindest dem Hauptmagneten 4 der Magnetresonanzvorrichtung 2 um eine Achse mittels der Positioniereinheit 42 gedreht. Die Achse verläuft hierbei durch eine Mitte des Aufnahmebereichs 12 im Wesentlichen parallel zu einer Einschubrichtung der Patientenliege 39, so dass die Linearbeschleunigereinheit 21 und die Targeteinheit 30 zusammen mit zumindest dem Hauptmagneten 4 der Magnetresonanzvorrichtung 2 um den Patienten 13 gedreht und in eine neue Bestrahlungsposition gebracht werden können.

Alternativ hierzu es denkbar, dass die Linearbeschleunigereinheit 21 und die Targeteinheit 30 der Strahlentherapievorrichtung 3 und der Hauptmagnet 4 der Magnetresonanzvorrichtung 2 fest positioniert sind und zu einer Variation des Bestrahlungswinkels die Patientenliege 39 zusammen mit dem Patienten 13 um eine Achse gekippt wird. Die Achse ist vorzugsweise von einer Längsachse der Patientenliege 39 gebildet.

In den FIG 3 bis 5 sind alternative Ausführungsbeispiele der Vorrichtung 1 dargestellt. Im Wesentlichen gleich bleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den FIG 1 und 2, wobei bezüglich gleich bleibender Bauteile, Merkmale und Funktionen auf die Beschreibung des Ausführungsbeispiels in den FIG 1 und 2 verwiesen wird.

In FIG 3 ist eine Vorrichtung 1 mit einer Kombination aus einer Magnetresonanzvorrichtung 2 und einer Strahlentherapievorrichtung 3 dargestellt. Die Strahlentherapievorrichtung 3 umfasst eine Linearbeschleunigereinheit 60 mit einem Vakuumgefäß 61, das innerhalb eines Hauptmagneten 4 der Magnetresonanzvorrichtung 2 angeordnet ist. Das Vakuumgefäß 61 der Linearbeschleunigereinheit 60 erstreckt sich hierbei durch den im Wesentlichen magnetfeldfreien Bereich 27 des Hauptmagneten 4. Ein Vakuumgefäß 62 des Hauptmagneten 4 ist zudem derart ausgestaltet, dass es das Vakuumgefäß 61 der Linearbeschleunigereinheit 60 zylinderförmig entlang einer Strahlrichtung des Partikelstrahls 20 umgibt. Innerhalb des Vakuumgefäßes 61 der Linearbeschleunigereinheit 60 ist ein Hohlraumresonator 63 der Linearbeschleunigereinheit 60 in einem außerhalb des Hauptmagneten 4 liegenden Bereich des Vakuumgefäßes 61 angeordnet. Entlang einer Strahlrichtung des Partikelstrahls 20 ist der Hohlraumresonator 63 vor dem Hauptmagneten 4 angeordnet. Alternativ hierzu ist auch eine Anordnung des Hohlraumresonators 63 innerhalb eines von dem Hauptmagneten 4 umschlossenen Bereichs denkbar.

Eine Targeteinheit 64 der Linearbeschleunigereinheit 60 ist innerhalb des Vakuumgefäßes 61 der Linearbeschleunigereinheit 60 angeordnet. Zudem ist die Targeteinheit 64 außerhalb des Vakuumgefäßes 62 der Magnetresonanzvorrichtung 2 angeordnet. Hierzu weist das Vakuumgefäß 62 der Magnetresonanzvorrichtung 2 eine Aussparung 65 auf, in der die Targeteinheit 64 angeordnet ist, wobei das Vakuumgefäß 61 der Linearbeschleunigereinheit 60 den Hauptmagneten 4 bis zu der Aussparung 64 entlang des im Wesentlichen magnetfeldfreien Bereichs 27 durchquert. Die Targeteinheit 64 umfasst eine Strahlumlenkungseinheit 66, ein Targetelement 67 und ein Kollimatorelement 68, wobei eine Funktionsweise der Targeteinheit 64 der Funktionsweise der Targeteinheit in den FIG 1 und 2 entspricht. Nach Verlassen des Kollimatorelements 68 tritt ein Gamma- und/oder Röntgenstrahl 36 in das Vakuumgefäß 62 des Hauptmagneten 4 ein und durchquert dieses im Wesentlichen senkrecht zur magnetischen Flussdichte 14 des Hauptmagnetfelds und tritt anschließend in den Aufnahmebereich 12 für eine Bestrahlung des Patienten ein.

In einer weiteren alternativen Ausgestaltung kann es ferner vorgesehen sein, dass die Targeteinheit 64 innerhalb des Vakuumgefäßes 62 des Hauptmagneten 4 angeordnet ist und somit auf die Aussparung 65 des Vakuumgefäßes 62 verzichtet werden kann.

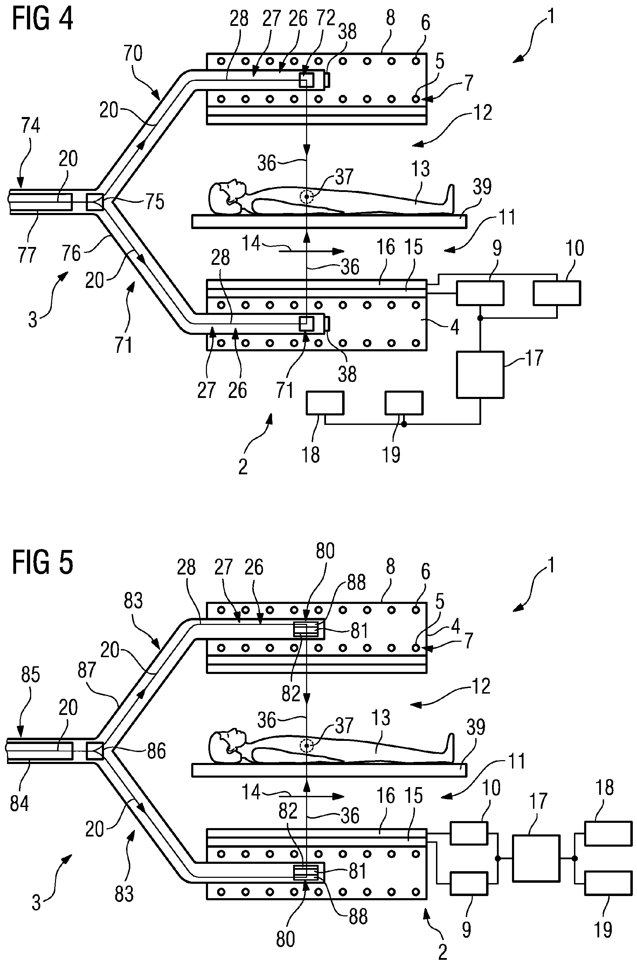

In FIG 4 ist eine weitere alternative Ausgestaltung der Vorrichtung 1 mit einer Kombination aus einer Magnetresonanzvorrichtung 2 und einer Strahlentherapievorrichtung 3 dargestellt. Die Strahlentherapievorrichtung 3 weist mehrere Strahlführungen 70, 71 für einen Elektronenstrahl auf, von denen zwei exemplarisch in FIG 4 dargestellt sind. Die Strahlführungen 70, 71 sind innerhalb eines Hauptmagneten 4 der Magnetresonanzvorrichtung 2 implementiert. Die einzelnen Strahlführungen 70, 71 verlaufen analog zu der Beschreibung zu FIG 1 zwischen zwei Magnetfeldspulen 5, 6 des Hauptmagneten 4 in einem im Wesentlichen feldfreien Bereich 27, wobei die Strahlführungen 70, 71 im Wesentlichen parallel zu einer Richtung einer magnetischen Flussdichte 14 des Hauptmagnetfelds ausgerichtet sind.

Ein Hohlraumresonator 77 einer Linearbeschleunigereinheit 74 ist außerhalb des Hauptmagneten 4 angeordnet, wobei die unterschiedlichen Strahlführungen 70, 71 von dem Hohlraumresonator 77 zunächst konusförmig, geradlinig auseinanderlaufen und bei Erreichen des Hauptmagneten 4 in den magnetfeldfreien Bereich 27 des Hauptmagneten 4 abbiegen. Innerhalb der Strahlführungen 70, 71 sind hierzu nicht näher dargestellte Unlenkmittel zur Strahlumlenkung entlang der Strahlführung 70, 71 des beschleunigten Partikelstrahls 28 angeordnet.

Zudem weist die Linearbeschleunigereinheit 74 eine Umlenkeinheit 75 auf, die den Elektronenstrahl nach dem Hohlraumresonator 77 in die ausgewählte Strahlführung 70, 71 lenkt. Des Weiteren sieht die Umlenkeinheit 75 der Linearbeschleunigereinheit 74 auch eine Strahlteilung des Partikelstrahls 28 vor, die den beschleunigten Partikelstrahl 28 entsprechend einer Anzahl der Strahlführungen 70, 71 teilt. Die daraus entstehenden Teilstrahlen können anschließend umgelenkt werden und in die jeweilige Strahlführung 70, 71 eingeführt werden, so dass eine gleichzeitige Bestrahlung aus unterschiedlichen Bestrahlungswinkeln und/oder Bestrahlungspositionen bewirkt wird. Für jede der Strahlführungen 70, 71 steht jeweils eine innerhalb der Magnetresonanzvorrichtung 2 angeordnete Targeteinheit 72, 73 der Strahlentherapievorrichtung 3 zur Verfügung.

Die Targeteinheiten 72, 73 sind innerhalb eines Vakuumgefäßes 76 der Linearbeschleunigereinheit 74 angeordnet. Zudem können die Targeteinheiten 72, 73 und das Vakuumgefäß 76 der Linearbeschleunigereinheit 74 mit den mehreren Strahlführungen 70, 71 analog zu der Beschreibung zu FIG 1 und 2 oder analog zu der Beschreibung zu FIG 3 angeordnet sein.

In FIG 5 ist eine weitere alternative Ausgestaltung der Vorrichtung 1 mit einer Kombination aus einer Magnetresonanzvorrichtung 2 und einer Strahlentherapievorrichtung 3 dargestellt. Die Strahlentherapievorrichtung 3 weist eine Targeteinheit 80 mit einem Targetelement 81 auf. Das Targetelement 81 ist hierbei von einem um einen Aufnahmebereich 12 der Magnetresonanzvorrichtung 2 umlaufenden Targetring gebildet und innerhalb des Hauptmagneten 4 angeordnet. Entlang der Strahlführung 83 ist vor dem Targetring und Umlenkring 88 zu einer Strahlumlenkung des Partikelstrahls 20 angeordnet. Zudem ist entlang einer Strahlführung 83 nach dem Targetring ein Kollimatorring 82 angeordnet, um eine an dem Targetring erzeugte Gamma- und/oder Röntgenstrahlung auf einen Behandlungsbereich 37 eines Patienten 13 zu fokussieren. Die Strahlführung 83 innerhalb des Hauptmagneten 4 ist zylindermantelförmig ausgebildet und verläuft entlang eines im Wesentlichen magnetfeldfreien Bereichs 27 des Hauptmagneten 4. Außerhalb des Hauptmagneten 4 verläuft die Strahlführung 83 von einem Hohlraumresonator 84 einer Linearbeschleunigereinheit 85 in Richtung des Hauptmagneten 4 in Form einer geradlinigen Kegelfläche nach außen. Nach dem Hohlraumresonator 84 ist eine Umlenkeinheit 86 der Linearbeschleunigereinheit 85 angeordnet, deren Funktionsweise analog zu der Beschreibung zu FIG 4 ist. Die Targeteinheit 81 ist innerhalb eines Vakuumgefäßes 87 der Linearbeschleunigereinheit 85 angeordnet. Zudem kann die Targeteinheit 81 und das Vakuumgefäß 87 der Linearbeschleunigereinheit 85 mit der Strahlführung 83 analog zu der Beschreibung zu FIG 1 und 2 oder analog zu der Beschreibung zu FIG 3 angeordnet sein.

Alternativ hierzu kann eine Zuführung eines Elektronenstrahls zu dem Targetring auch über mehrere innerhalb eines Hauptmagneten 4 implementierte Strahlführungen analog zu der Beschreibung zu FIG 4 erfolgen.

Alternativ zu den in den FIG 1 bis 5 beschriebenen Ausführungsbeispielen kann zudem auf eine Strahlumlenkungseinheit innerhalb der Targeteinheit verzichtet werden. Hierbei ist jedoch ein von einem Reflexionstarget gebildetes Targetelements erforderlich, bei dem der einfallende Elektronenstrahl und der ausgehende Gamma- und/oder Röntgenstrahl einen Winkel von im Wesentlichen ungleich 0° einschließen.

Zudem kann alternativ zu den in den FIG 1 bis 5 beschriebenen Ausführungsbeispielen lediglich eine Strahlumlenkungseinheit und/oder ein Targetelement der Targeteinheit innerhalb des Vakuumgefäßes der Linearbeschleunigereinheit oder innerhalb des Vakuumgefäßes des Hauptmagneten angeordnet sein. Ein Kollimatorelement der Targeteinheit kann hierbei außerhalb des Vakuumgefäßes der Linearbeschleunigereinheit oder des Vakuumgefäßes des Hauptmagneten angeordnet sein.

Weiterhin kann alternativ zu den in den FIG 1 bis 5 beschriebenen Ausführungsbeispielen ein herkömmlicher supraleitender Magnet innerhalb des Hauptmagneten angeordnet sein, wobei eine Kühlung von Windungen der einzelnen Magnetfeldspulen in einem Heliumbad erfolgt. Hierbei würde der Elektronenstrahl stets in einem zu dem Vakuumgefäß des Hauptmagneten separat ausgebildeten Vakuumgefäß erfolgen. Zudem kann der Hauptmagnet auch von einem Niederfeldmagneten gebildet sein oder ein Niedermagnetfeld erzeugen.

## Patentansprüche

1. Vorrichtung mit einer Kombination aus einer Magnetresonanzvorrichtung (2), die zumindest einen Hauptmagneten (4) zu einem Erzeugen eines Hauptmagnetfelds in einem Untersuchungsraum (11) für eine Magnetresonanzmessung aufweist, und einer Strahlentherapievorrichtung (3), die zu einer Erzeugung eines Partikelstrahls (20) vorgesehen ist und die eine Strahlführung (44, 70, 71, 83) aufweist wobei die Magnetresonanzvorrichtung (2) einen im Wesentlichen magnetfeldfreien Bereich (27) aufweist und die Strahlführung (44, 70, 71, 83) des Partikelstrahls (20) entlang des im Wesentlichen magnetfeldfreien Bereichs (27) verläuft,
**dadurch gekennzeichnet, dass** die Strahlführung (44, 70, 71, 83) des Partikelstrahls (20) zumindest teilweise innerhalb des Hauptmagneten (4) entlang des im Wesentlichen magnetfeldfreien Bereichs (27) verläuft,
wobei der Hauptmagnet (4) eine erste Magnetfeldspule (5) zum Erzeugen eines ersten Magnetfelds und zumindest eine zweite Magnetfeldspule (6) zum Erzeugen eines zweiten Magnetfelds aufweist, so dass der im Wesentlichen magnetfeldfreie Bereich (27) innerhalb des Hauptmagneten (4) vorliegt.

2. Vorrichtung nach Anspruch 1,
**dadurch gekennzeichnet, dass** die Strahlführung (44, 70, 71, 83) zumindest teilweise zwischen den beiden Magnetfeldspulen (5, 6) verläuft.

3. Vorrichtung nach einem der vorhergehenden Ansprüche, **dadurch gekennzeichnet, dass** die Strahlführung (44, 70, 71, 83) zumindest teilweise parallel zu einer Richtung und/oder einer Orientierung einer magnetischen Flussdichte (14) des Hauptmagnetfelds verläuft.

4. Vorrichtung nach einem der vorhergehenden Ansprüche, **dadurch gekennzeichnet, dass** der Hauptmagnet (4) zumindest zwei einzelne Magnete aufweist, die zusammen in einem Vakuumgefäß (8, 62) angeordnet sind.

5. Vorrichtung nach einem der vorhergehenden Ansprüche, **dadurch gekennzeichnet, dass** die Strahlführung (44, 70, 71, 83) entlang einer zumindest teilweise gekrümmten Trajektorie (28) innerhalb des Hauptmagneten (4) verläuft.

6. Vorrichtung nach einem der vorhergehenden Ansprüche, **dadurch gekennzeichnet, dass** die Strahlentherapievorrichtung (3) eine Beschleunigereinheit aufweist, die zumindest teilweise innerhalb der Magnetresonanzvorrichtung (2) angeordnet ist.

7. Vorrichtung nach Anspruch 6,
**dadurch gekennzeichnet, dass** die Beschleunigereinheit von einer Linearbeschleunigereinheit (21) gebildet ist.

8. Vorrichtung zumindest nach Anspruch 6,
**dadurch gekennzeichnet, dass** die Beschleunigereinheit zumindest teilweise innerhalb des Hauptmagneten (4) angeordnet ist.

9. Vorrichtung zumindest nach Anspruch 6,
**dadurch gekennzeichnet, dass** die Beschleunigereinheit ein zu einem Vakuumgefäß (8, 62) des Hauptmagneten (4) separates Vakuumgefäß (24, 61, 76, 87) aufweist.

10. Vorrichtung zumindest nach Anspruch 6,
**dadurch gekennzeichnet, dass** die Beschleunigereinheit zumindest teilweise innerhalb eines Vakuumgefäßes (8, 62) des Hauptmagneten (4) angeordnet ist.

11. Vorrichtung nach einem der vorhergehenden Ansprüche, **dadurch gekennzeichnet, dass** der Hauptmagnet (4) ein Vakuumgefäß (8, 62) mit zumindest einem Eintritt- und/oder Austrittsfenster (29) aufweist.

12. Vorrichtung nach einem der vorhergehenden Ansprüche, **dadurch gekennzeichnet, dass** die Strahlentherapievorrichtung (3) zumindest ein Targetelement (31, 67, 81) aufweist, das innerhalb eines Vakuumgefäßes (8, 24, 61, 62, 76, 87) der Strahlentherapievorrichtung (3) und/oder des Hauptmagneten (4) angeordnet ist.

13. Vorrichtung nach einem der vorhergehenden Ansprüche, **dadurch gekennzeichnet, dass** die Strahlentherapievorrichtung (3) zumindest ein Targetelement (81) aufweist, das von einem um einen Aufnahmebereich (12) der Magnetresonanzvorrichtung (2) umlaufenden Targetring gebildet ist.

14. Vorrichtung nach einem der vorhergehenden Ansprüche, **dadurch gekennzeichnet, dass** die Strahlentherapievorrichtung (3) zumindest ein Kollimatorelement (32, 68, 82) aufweist, das innerhalb eines Vakuumgefäßes (8, 24, 61, 62, 76, 87) der Strahlentherapievorrichtung (3) und/oder des Hauptmagneten (4) angeordnet ist.

15. Vorrichtung nach einem der vorhergehenden Ansprüche, **dadurch gekennzeichnet, dass** die Strahlentherapievorrichtung (3) zumindest ein Kollimatorelement (8, 24, 61, 62, 76, 87) aufweist, das um zumindest eine Achse und/oder entlang zumindest einer Achse bewegbar angeordnet ist.

16. Vorrichtung nach einem der vorhergehenden Ansprüche, **dadurch gekennzeichnet, dass** die Strahlentherapievorrichtung (3) zumindest zwei Strahlführungen (70, 71) für den Partikelstrahl (20) aufweist und die zwei Strahlführungen (70, 71) innerhalb der Magnetresonanzvorrichtung (2) angeordnet sind.

17. Vorrichtung nach Anspruch 16,
**dadurch gekennzeichnet, dass** die zumindest zwei Strahlführungen (70, 71) für den Partikelstrahl (20) zumindest teilweise innerhalb des Hauptmagneten (4) angeordnet sind.

18. Vorrichtung nach einem der vorhergehenden Ansprüche, **dadurch gekennzeichnet, dass** die Strahlführung (44 ,70, 71, 83) zusammen mit der zumindest teilweisen Magnetresonanzvorrichtung (2) entlang wenigstens einer Richtung verschiebbar angeordnet ist.

19. Vorrichtung nach einem der vorhergehenden Ansprüche, **dadurch gekennzeichnet, dass** die Strahlführung (44, 70, 71, 83) zusammen mit der zumindest teilweisen Magnetresonanzvorrichtung (2) um zumindest eine Achse drehbar angeordnet ist.

20. Vorrichtung nach einem der vorhergehenden Ansprüche, **dadurch gekennzeichnet**, die Magnetresonanzvorrichtung (2) eine Patientenliege (39) zu einem Einführen eines Patienten (13) in einen Aufnahmebereich (12) aufweist und die Patientenliege (39) entlang zumindest zwei Richtungen verschiebbar angeordnet ist.

21. Vorrichtung nach einem der vorhergehenden Ansprüche, **dadurch gekennzeichnet, dass** der Partikelstrahl (20) und ein aus dem Partikelstrahl (20) erzeugter Gamma- und/oder Röntgenstrahl (36) im Wesentlichen orthogonal zueinander ausgerichtet sind.

22. Vorrichtung nach einem der vorhergehenden Ansprüche, **dadurch gekennzeichnet, dass** die Magnetresonanzvorrichtung (2) von einer Hochfeldmagnetresonanzvorrichtung gebildet ist.

## Claims

1. Apparatus having a combined magnetic resonance apparatus (2), which features at least one main magnet (4) for generating a main magnetic field in an examination space (11) for a magnetic resonance measurement, and a radiation therapy apparatus (3), which is provided to generate a particle beam (20) and which features a beam guide (44, 70, 71, 83), wherein the magnetic resonance apparatus (2) features an essentially magnetic-field-free region (27) and the beam guide (44, 70, 71, 83) for the particle beam (20) runs along the essentially magnetic-field-free region (27),
**characterised in that** the beam guide (44, 70, 71, 83) of the particle beam (20) runs at least partially within the main magnet (4) along the essentially magnetic-field-free region (27),
wherein the main magnet (4) features a first magnetic field coil (5) for generating a first magnetic field and at least one second magnetic field coil (6) for generating a second magnetic field, so that the essentially magnetic-field-free region (27) is produced within the main magnet (4).

2. Apparatus according to claim 1,
**characterised in that** the beam guide (44, 70, 71, 83) runs at least partially between the two magnetic field coils (5, 6).

3. Apparatus according to one of the preceding claims, **characterised in that** the beam guide (44, 70, 71, 83) runs at least partially parallel to a direction and/or orientation of a magnetic flux density (14) of the main magnetic field.

4. Apparatus according to one of the preceding claims, **characterised in that** the main magnet (4) features at least two individual magnets, which are disposed together in a vacuum vessel (8, 62).

5. Apparatus according to one of the preceding claims, **characterised in that** the beam guide (44, 70, 71, 83) runs along an at least partially curved trajectory (28) within the main magnet (4).

6. Apparatus according to one of the preceding claims, **characterised in that** the radiation therapy apparatus (3) features an accelerator unit, which is disposed at least partially within the magnetic resonance apparatus (2).

7. Apparatus according to claim 6,
**characterised in that** the accelerator unit is formed by a linear accelerator unit (21).

8. Apparatus at least according to claim 6,
**characterised in that** the accelerator unit is disposed at least partially within the main magnet (4).

9. Apparatus at least according to claim 6,
**characterised in that** the accelerator unit features a vacuum vessel (24, 61, 76, 87) separate from a vacuum vessel (8, 62) of the main magnet (4).

10. Apparatus at least according to claim 6,
**characterised in that** the accelerator unit is disposed at least partially within a vacuum vessel (8, 62) of the main magnet (4).

11. Apparatus according to one of the preceding claims, **characterised in that** the main magnet (4) features a vacuum vessel (8, 62) with at least one entry and/or exit window (29).

12. Apparatus according to one of the preceding claims, **characterised in that** the radiation therapy apparatus (3) features at least one target element (31, 67, 81), which is disposed within a vacuum vessel (8, 24, 61, 62, 76, 87) of the radiation therapy apparatus (3) and/or the main magnet (4).

13. Apparatus according to one of the preceding claims, **characterised in that** the radiation therapy apparatus (3) features at least one target element (81), which is formed by a target ring around a receiving region (12) of the magnetic resonance apparatus (2).

14. Apparatus according to one of the preceding claims, **characterised in that** the radiation therapy apparatus (3) features at least one collimator element (32, 68, 82), which is disposed within a vacuum vessel (8, 24, 61, 62, 76, 87) of the radiation therapy apparatus (3) and/or the main magnet (4).

15. Apparatus according to one of the preceding claims, **characterised in that** the radiation therapy apparatus (3) features at least one collimator element (8, 24, 61, 62, 76, 87), which is disposed in such a manner that it can be moved about at least one axis and/or along at least one axis.

16. Apparatus according to one of the preceding claims, **characterised in that** the radiation therapy apparatus (3) features at least two beam guides (70, 71) for the particle beam (20) and the two beam guides (70, 71) are disposed within the magnetic resonance apparatus (2).

17. Apparatus according to claim 16,
**characterised in that** the at least two beam guides (70, 71) for the particle beam (20) are disposed at least partially within the main magnet (4).

18. Apparatus according to one of the preceding claims, **characterised in that** the beam guide (44 ,70, 71, 83) is disposed in such a manner that it can be displaced together with the at least partial magnetic resonance apparatus (2) along at least one direction.

19. Apparatus according to one of the preceding claims, **characterised in that** the beam guide (44, 70, 71, 83) is disposed in such a manner that it can be rotated together with the at least partial magnetic resonance apparatus (2) about at least one axis.

20. Apparatus according to one of the preceding claims, **characterised in that** the magnetic resonance apparatus (2) features a patient couch (39) for moving a patient (13) into a receiving region (12) and the patient couch (39) is disposed in such a manner that it can be displaced along at least two directions.

21. Apparatus according to one of the preceding claims, **characterised in that** the particle beam (20) and a gamma and/or x-ray beam (36) generated from the particle beam (20) are aligned essentially orthogonally to one another.

22. Apparatus according to one of the preceding claims, **characterised in that** the magnetic resonance apparatus (2) is formed by a high field magnetic resonance apparatus.

## Revendications

1. Dispositif comprenant une combinaison d'un dispositif ( 2 ) à résonance magnétique qui a, pour une mesure de résonance magnétique, au moins un aimant ( 4 ) principal de production d'un champ magnétique principal dans un espace ( 11 ) d'analyse et d'un dispositif ( 3 ) de thérapie par rayonnement, qui est prévu pour une production d'un faisceau ( 20 ) de particules et qui a un guidage ( 44, 70, 71, 83 ) de faisceau, le dispositif ( 2 ) à résonance magnétique ayant une partie ( 27 ) sensiblement sans champ magnétique et le guidage ( 44, 70, 71, 83 ) du faisceau ( 20 ) de particules s'étendant le long de la partie ( 27 ) sensiblement sans champ magnétique,
**caractérisé en ce que** le guidage ( 44, 70, 71, 83 ) du faisceau ( 20 ) de particules s'étend au moins en partie au sein de l'aimant ( 4 ) principal, le long de la partie ( 27 ) sensiblement sans champ magnétique,
dans lequel l'aimant ( 4 ) principal a une première bobine ( 5 ) de champ magnétique pour la production d'un premier champ magnétique et au moins une deuxième bobine ( 6 ) de champ magnétique pour la production d'un deuxième champ magnétique, de manière à ce que la partie ( 27 ) sensiblement sans champ magnétique se trouve au sein de l'aimant ( 4 ) principal.

2. Dispositif suivant la revendication 1,
**caractérisé en ce que** le guidage ( 44, 70, 71, 83 ) du faisceau s'étend au moins en partie entre les deux bobines ( 5, 6 ) de champ magnétique.

3. Dispositif suivant l'une des revendications précédentes, **caractérisé en ce que** le guidage ( 44, 70, 71, 83 ) du faisceau s'étend au moins en partie parallèlement à une direction/ou à une orientation d'une densité ( 14 ) de flux magnétique du champ magnétique principal.

4. Dispositif suivant l'une des revendications précédentes, **caractérisé en ce que** l'aimant ( 4 ) principal a au moins deux aimants individuels qui sont disposés ensemble dans un récipient ( 8, 62 ) sous vide.

5. Dispositif suivant l'une des revendications précédentes, **caractérisé en ce que** le guidage ( 44, 70, 71, 83 ) du faisceau s'étend au sein de l'aimant ( 4 ) principal suivant une trajectoire ( 28 ) au moins en partie courbée.

6. Dispositif suivant l'une des revendications précédentes, **caractérisé en ce que** le dispositif ( 3 ) de thérapie par rayonnement a une unité d'accélérateur disposée au moins en partie dans le dispositif ( 2 ) à résonance magnétique.

7. Dispositif suivant la revendication 6,
**caractérisé en ce que** l'unité d'accélérateur est formée d'une unité ( 21 ) d'accélérateur linéaire.

8. Dispositif suivant au moins la revendication 6, **caractérisé en ce que** l'unité d'accélérateur est disposée au moins en partie dans l'aimant ( 4 ) principal.

9. Dispositif suivant au moins la revendication 6, **caractérisé en ce que** l'unité d'accélérateur a un récipient ( 24, 61, 76, 87 ) sous vide distinct d'un récipient ( 8, 62 ) sous vide de l'aimant ( 4 ) principal.

10. Dispositif suivant au moins la revendication 6, **caractérisé en ce que** l'unité d'accélérateur est disposée au moins en partie à l'intérieur d'un récipient ( 8, 62 ) sous vide de l'aimant ( 4 ) principal.

11. Dispositif suivant l'une des revendications précédentes, **caractérisé en ce que** l'aimant ( 4 ) principal a un récipient ( 8, 62 ) sous vide ayant au moins un hublot ( 29 ) d'entrée/ou de sortie.

12. Dispositif suivant l'une des revendications précédentes, **caractérisé en ce que** le dispositif ( 3 ) de thérapie par rayonnement a au moins un élément ( 31, 67, 81 ) cible, qui est disposé à l'intérieur d'un récipient ( 8, 24, 61, 62, 76, 87 ) sous vide, du dispositif ( 3 ) de thérapie par rayonnement et/ou de l'aimant ( 4 ) principal.

13. Dispositif suivant l'une des revendications précédentes, **caractérisé en ce que** le dispositif ( 3 ) de thérapie par rayonnement a au moins un élément ( 81 ) cible, qui est formé d'un anneau cible entourant une partie ( 12 ) de réception du dispositif ( 2 ) à résonance magnétique.

14. Dispositif suivant l'une des revendications précédentes, **caractérisé en ce que** le dispositif ( 3 ) de thérapie par rayonnement a au moins un élément ( 32, 68, 82 ) de collimateur, qui est disposé à l'intérieur d'un récipient ( 8, 24, 61, 62, 76, 87 ) sous vide, du dispositif ( 3 ) de thérapie par rayonnement et/ou de l'aimant ( 4 ) principal.

15. Dispositif suivant l'une des revendications précédentes, **caractérisé en ce que** le dispositif ( 3 ) de thérapie par rayonnement a au moins un élément ( 8, 24, 61, 62, 76, 87 ) de collimateur, qui est monté mobile autour d'au moins un axe et/ou le long d'au moins un axe.

16. Dispositif suivant l'une des revendications précédentes, **caractérisé en ce que** le dispositif ( 3 ) de thérapie par rayonnement a au moins deux guidages ( 70, 71 ) du faisceau ( 20 ) de particules et les deux guidages ( 70, 71 ) du faisceau sont disposés à l'intérieur du dispositif ( 2 ) à résonance magnétique.

17. Dispositif suivant la revendication 16,
**caractérisé en ce que** les au moins deux guidages ( 70, 71 ) du faisceau ( 20 ) de particules sont disposés au moins en partie à l'intérieur de l'aimant ( 4 ) principal.

18. Dispositif suivant l'une des revendications précédentes, **caractérisé en ce que** le guidage ( 44, 70, 71, 83 ) du faisceau est monté coulissant avec le, au moins en partie, dispositif ( 2 ) à résonance magnétique, suivant au moins une direction.

19. Dispositif suivant l'une des revendications précédentes, **caractérisé en ce que** le guidage ( 44, 70, 71, 83 ) du faisceau est monté tournant autour d'au moins un axe, ensemble avec le, au moins en partie, dispositif ( 2 ) à résonance magnétique.

20. Dispositif suivant l'une des revendications précédentes, **caractérisé en ce que** le dispositif ( 2 ) à résonance magnétique a une couchette ( 39 ) de patient, pour l'entrée d'un patient ( 13 ) dans une partie ( 12 ) d'enregistrement et la couchette ( 39 ) du patient est montée coulissante suivant au moins deux directions.

21. Dispositif suivant l'une des revendications précédentes, **caractérisé en ce que** le faisceau ( 20 ) de particules et un faisceau ( 36 ) gamma et/ou de rayon X produit à partir du faisceau ( 20 ) de particules sont dirigés sensiblement orthogonalement l'un à l'autre.

22. Dispositif suivant l'une des revendications précédentes, **caractérisé en ce que** le dispositif ( 2 ) à résonance magnétique est formé d'un dispositif à résonance magnétique à champ intense.
